(19) Europäisches Patentamt
European Patent Office
Office européen des brevets

(11) EP 3 839 056 A1

(12) # EUROPEAN PATENT APPLICATION

(43) Date of publication:
**23.06.2021 Bulletin 2021/25**

(21) Application number: **19218701.1**

(22) Date of filing: **20.12.2019**

(51) Int Cl.:
***C12P 23/00*** *(2006.01)*
***C12N 9/10*** *(2006.01)*
***C12N 15/01*** *(2006.01)*
***C12Q 1/6895*** *(2018.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **NextFerm Technologies Ltd.**
**2069208 Yokneam Illit (IL)**

(72) Inventors:
- **Shemesh, Paz**
**1246101 Tiberias (IL)**
- **Cohen, Tzafra**
**3452718 Haifa (IL)**
- **Lifshitz, Yael**
**3094206 Zichron Yaakov (IL)**
- **Khutorian, Marina**
**3681142 Nesher (IL)**
- **Harari, Yaniv**
**2062417 Yokneam Illit (IL)**

(74) Representative: **Greiner, Elisabeth**
**df-mp Dörries Frank-Molnia & Pohlman**
**Patentanwälte Rechtsanwälte PartG mbB**
**Theatinerstraße 16**
**80333 München (DE)**

(54) **ASTAXANTHIN OVER-PRODUCING STRAINS OF PHAFFIA RHODOZYMA**

(57) The present invention relates to novel yeast strains of *Phaffia rhodozyma* which produce high amounts of carotenoids, in particular high amounts astaxanthin. These novel strains are capable of producing increasing amounts of carotenoids in the presence of increasing concentrations of carbon source.

EP 3 839 056 A1

Printed by Jouve, 75001 PARIS (FR)

## Description

### FIELD OF THE INVENTION

The present invention relates to novel yeast strains of *Phaffia rhodozyma* which produce high amounts of carotenoids, in particular high amounts of astaxanthin. These novel strains are capable of producing increasing amounts of carotenoids in the presence of increasing concentrations of carbon source.

### BACKGROUND OF THE INVENTION

Astaxanthin (3,3'-dihydroxy-β, β'-carotene-4,4'-dione) is a naturally-occurring lipid-soluble red oxycarotenoid pigment that is found in certain marine plants, crustaceans, fish and yeast. In commercial aquaculture, astaxanthin is often added to the diet of salmonids and crustaceans to impart in their flesh the distinctive pink colouration found in indigenous species. Imparting this distinctive pink colouration is believed to be important in encouraging consumer acceptance of salmonids and crustaceans produced through aquaculture.

The planar structure of astaxanthin is shown below:

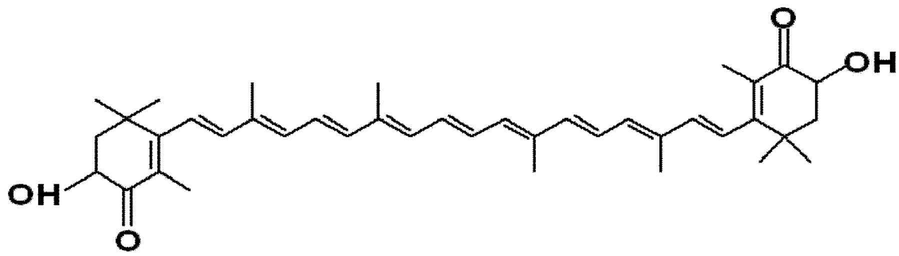

Astaxanthin is characterized by the presence of oxygen molecules in its structure. The astaxanthin molecule has two asymmetric carbons located at the 3, 3' positions of the β-ionone ring with a hydroxyl group (-OH) on either end of the molecule. Astaxanthin is composed of hydroxyl and keto moieties on both ends. Its lipophilic and hydrophilic properties allow it to align in the phospholipid bilayer of the cell membrane (Yang 2013, J Hum Nutr Food Sci 1:1003, Ambati 2014, Mar Drugs. 2014 Jan; 12(1)). The red pigment colour is due to the conjugated double bonds at the centre of the compound. This type of conjugated double bond acts as a strong antioxidant by donating electrons and reacting with free radicals to convert them into more stable products and terminating free radical chain reactions in a wide variety of living organisms.

Astaxanthin has been widely studied and it has been demonstrated that it has numerous clinical health benefits such as antioxidation, anti-inflammation, being anti-diabetic, in sports nutrition (mitigating effects on endurance, sports performance and heat stress), prevention of CVD, and promotion of healthy brain, eyes and skin (Yang 2013, J Hum Nutr Food Sci 1:1003; Ambati 2014, Mar Drugs. 2014 Jan; 12(1)). Astaxanthin's antioxidant activity has been demonstrated in several studies and it has been reported that it has up to several-fold stronger free radical antioxidant activity than vitamin E and β-carotene (Kurashge et.al., 1990, Shimidzu et.al., 1996). The antioxidant properties of astaxanthin are believed to have a key role in several other properties such as protection against UV-light photooxidation, cancer, *Helicobacter pylori* infection, aging and age-related diseases, and in promotion of the immune response, and liver, heart, joint and prostate health (Guerin, Huntley and Olaizola, 2003).

Sources of astaxanthin are in great demand by the aquaculture industry, particularly as astaxanthin is an expensive feed ingredient, and for human consumption. The natural sources of astaxanthin for industrial use are microalgae (*Haematococcus pluvialis*), the yeast *Phaffia rhodozyma* and marine bacteria *Paracoccus carotinifaciens.* The aquaculture industry continues to expand and the production of Atlantic salmon also has increased in recent years. Since farmed salmon, trout and crustacea are not fed stable diets of natural astaxanthin sources such as shrimp and krill, their astaxanthin consumption must be derived from the feed consumed. Without the addition of astaxanthin to their diet, farm grown salmon are white instead of orange-pink in colour. Synthetic astaxanthin is the main carotenoid used worldwide in the aquaculture industry. Synthetic astaxanthin consists of a mixture 1:2:1 of isomers (3S, 3S), (3R, 3S), and (3R, 3R), respectively. However, the growing consumers' demand for natural foods without the use of synthetic dyes is the underlying driver for major efforts to improve astaxanthin production from biological sources instead of synthetic ones. Phaffia yeast has been proven to be a reliable source of astaxanthin, but current solutions are extremely costly.

The yeast *Phaffia rhodozyma* was first isolated from slime fluxes of birch trees in colder regions, such as in Russia, Chile, Finland, Japan and the United States, in the late 1960s by Herman J. Phaff. This yeast reproduced

vegetatively by budding and later a sexual state of *Phaffia rhodozyma* was defined and was named *Xanthophyllomyces dendrorhous* (XDEN). The yeast *Phaffia rhodozyma* is of basidiomycetous origin. Criteria used in identifying *Phaffia rhodozyma* as basidiomycetous include its ability to synthesize carotenoids, of which astaxanthin is the principal component; its multi-layered cell wall structure and mode of bud formation and its ability to ferment several sugars; a property not shared by any other carotenoid-producing yeast.

The wild-type strain of *Phaffia rhodozyma* produces low levels of astaxanthin typically 100 to 300 parts per million (ppm) of the yeast dry mass and hence they are not a practical or economical source (Torrissen, Hardy and Shearer, 1989). Mutant strains of *Phaffia rhodozyma* have been described as being capable of producing high levels of astaxanthin under certain growth conditions such as low glucose levels in the growth media; see for example, US5356809, US5922560, Barredo 2012, An et al 1989, Jeong-Hwan 2004, and Baeza 2010. Astaxanthin over-producing *Phaffia rhodozyma* strains with over 5000 μg astaxanthin per g yeast dry mass can be obtained through mutagenesis of the wild-type strain. However, due to the increasing needs for astaxanthin in the food, nutrition and health industries, there exists a need for further strains of *Phaffia rhodozyma* which produce even higher levels of astaxanthin under a range of growth conditions, in particular in the presence of changing amounts or higher amounts of carbon source, and also when the yeast is cultivated at high biomass concentrations. The novel *Phaffia rhodozyma* strains of the invention fulfil these needs, and produce very high levels of astaxanthin and are amenable to industrial-scale fermentation.

## SUMMARY OF THE INVENTION

The invention provides astaxanthin over-producing strains which produce exceptionally high levels of carotenoids, such as astaxanthin. Remarkably, these strains are able to produce increased amounts of carotenoids in the presence of 1-10% carbon source, making them particularly amenable to industrial-scale fermentation in which fluctuations in carbon source concentrations will typically occur. Such strains can therefore provide increased efficiency and increased yield of carotenoid production, in particular of astaxanthin production. The strains of the invention therefore have application for the industrial production of carotenoids, in particular astaxanthin, both for the aquaculture industry and for human consumption.

Accordingly, the invention provides yeast strains of *Phaffia rhodozyma,* wherein the yeast strains are *inter alia* characterized in that they are capable of producing a greater amount of carotenoid when cultivated in a yeast growth medium comprising at least 3% (w/v) carbon source compared to when cultivated in a yeast growth medium comprising 1% (w/v) carbon source.

## DESCRIPTION OF THE INVENTION

### Definitions

The term "NCYC" stands for "National Collection of Yeast Cultures", which is an International Depository Authority under the Budapest Treaty accepting yeast strain deposits for patent purposes.

The term "CBS" stands for "Centraalbureau voor Schimmelcultures", which is an International Depository Authority under the Budapest Treaty accepting yeast strain deposits for patent purposes.

As used herein, amounts of carotenoid (e.g., astaxanthin) are expressed as the mass of carotenoid per mass of dry yeast, for example: μg astaxanthin per g dry yeast or dry mass (d.m.).

The total amount of carotenoid is abbreviated herein as "TC".

A "carbon source" according to the present invention can be a monosaccharide, a disaccharide, a trisaccharide, or an oligosaccharide, for example, glucose, fructose, sucrose, maltose, raffinose, xylose, corn syrup, glycerol, succinate, pyruvate or combinations thereof. Preferably, the carbon source is glucose, fructose, sucrose, maltose, raffinose, xylose, or a combination thereof.

As used herein, "yeast growth medium" is a selective growth medium of acidic pH which permits the growth of yeast, while deterring growth of bacteria and other acid-intolerant organisms. An example of such a medium is yeast and mold "YM" medium, which is the standard yeast agar/broth as known in the art. Such a medium typically contains a carbon source, a nitrogen source and salts and it may contain peptone (a water-soluble mixture of polypeptides and amino acids formed by the partial hydrolysis of protein), trace elements and yeast extract. An exemplary YM medium composition can contain yeast extract 3 g/L, malt extract 3 g/L, peptone 5 g/L, and a carbon source 10 to 50 g/L.

As used herein, percentages of carbon source are weight/volume.

As used herein, the term "v/v" means volume/volume and the term "w/w" means weight/weight.

As used herein, a "mutation" can comprise a deletion of nucleotides, a substitution of nucleotides, or an insertion of nucleotides (which may result in a frame-shift) in the protein-encoding sequence.

As used herein, a "point mutation" is a substitution of a nucleotide for another nucleotide in a nucleotide sequence, for example in a protein-encoding sequence, in intronic sequences or in upstream/downstream sequence

regions. Preferably, said point mutation is in a protein-encoding sequence and results in the encoding of a different amino acid.

*Phaffia Rhodozyma* Strains

The invention provides a yeast strain of *Phaffia rhodozyma,* wherein the yeast strain is characterized in that it is capable of producing a greater amount of carotenoid when cultivated in a yeast growth medium comprising at least 3% (w/v) carbon source compared to when cultivated in a yeast growth medium comprising 1% (w/v) carbon source. For example, the yeast strain is capable of producing a greater amount of carotenoid when cultivated in a yeast growth medium comprising from about 3% (w/v) to about 10% (w/v), or from about 3% (w/v) to about 8% (w/v), or from about 3% (w/v) to about 5% (w/v), or about 3.5% (w/v), or about 4% (w/v), or about 4.5% (w/v), or about 5% (w/v), or about 6% (w/v), or about 7% (w/v), or about 8% (w/v), carbon source compared to when cultivated in a yeast growth medium comprising 1% (w/v) carbon source.

Preferably, the yeast strain of the invention is capable of producing a greater amount of carotenoid when cultivated in a yeast growth medium comprising from about 3% (w/v) to about 8% (w/v), or from about 3% (w/v) to about 5% (w/v), or about 3.5% (w/v), about 4% (w/v), about 4.5% (w/v) or about 5% (w/v) carbon source compared to when cultivated in a yeast growth medium comprising 1% (w/v) carbon source.

The yeast strain of the invention may comprise a genome comprising at least one, at least two, at least three, at least four, at least five, or at least six of i) to vi) defined in the following:

i) a point mutation at position 2028187 in scaffold 69, at the upstream region of gene XDEN_04715 (i.e., the point mutation referred to in SEQ ID NO:5 according to Table 20);

ii) a point mutation at position 1540450 in scaffold 79 at gene XDEN_05955 (i.e., the point mutation referred to in SEQ ID NO:4 according to Table 20);

iii) two-point mutations at positions 318703 and 318738 in scaffold 242, upstream to gene XDEN_00738 (i.e., the point mutations referred to in SEQ ID NO:1 according to Table 20);

iv) two-point mutations at positions 855 and 1020 in scaffold 88, downstream to gene XDEN_06229 (i.e., the point mutations referred to in SEQ ID NO:2 according to Table 20);

v) a deletion of two bases immediately following position 213879 in scaffold 162, located at the gene XDEN_00195 (i.e., the mutation referred to in SEQ ID NO:3 according to Table 20); and

vi) two-point mutations at positions 84078 and 84206 in scaffold 24, at the gene XDEN_01573 (i.e., the point mutations referred to in SEQ ID NO:6 according to Table 20).

In some embodiments, the genome comprises all of the mutations as defined in i) to vi).

Preferably, the point mutation(s) is (are) present in all alleles of said gene(s).

In a preferred embodiment, the yeast strain of the invention may comprise a genome comprising a point mutation at position 1540450 in scaffold 79 at gene XDEN_05955 named *crtE.* Preferably, the point mutation at position 1540450 in scaffold 79 is a G to A (guanine to adenine) change, resulting in an amino acid exchange of proline to serine (at gene XDEN_05955 which is encoded on the complementary reverse strand (codon CCG → TCG base substitution)).

In some embodiments, a point mutation in scaffold 79 at gene XDEN_05955 named *crtE* results in an alteration of *crtE* function. Function of *crtE* (also known as geranylgeranyl pyrophosphate synthetase) can for example be measured by quantification of geranylgeranyl pyrophosphate (GGPP) amounts in extracts of *Phaffia rhodozyma.*

In one embodiment, the yeast strain of the invention may comprise a genome comprising a point mutation at position 2028187 in scaffold 69, at the upstream region of gene XDEN_04715 named *erg20.*

In some embodiments, a point mutation in scaffold 69 at gene XDEN_04715 named *erg20* results in an alteration of *erg20* function. Function of *erg20* (also known as farnesyl pyrophosphate synthase) can for example be measured by overexpressing the mutated gene in cell culture (e.g., in yeast or in *E.coli*), isolating protein extracts from transformed cells and performing farnesyl pyrophosphate synthase activity assays *in vitro* with said protein extracts. Overexpression of wild-type *erg20* can be used as a positive control, and non-transformed or non-induced cultures can be used as a negative control.

In preferred embodiments, the yeast strain of the invention comprises a genome comprising a point mutation at position 1540450 in scaffold 79 at gene XDEN_05955 named *crtE,* and in addition a point mutation at position 2028187 in scaffold 69 in the upstream region of gene XDEN_04715 named *erg20.*

The yeast strain of the invention may comprise a genome comprising two-point mutations at positions 318703 and 318738 in scaffold 242, upstream to gene XDEN_00738.

The yeast strain of the invention may comprise a genome comprising two-point mutations at positions 855 and 1020 in scaffold 88, downstream to gene XDEN_06229.

The yeast strain of the invention may comprise a genome comprising a deletion of two bases immediately following position 213879 in scaffold 162, at the gene XDEN_00195.

The yeast strain of the invention may comprise a genome comprising two-point mutations at positions 84078 and 84206 in scaffold 24, at the gene XDEN_01573. Whereas the mutation in position 84078 is defined as an intronic mutation, the mutation in position 84206 is an exonic mutation which leads to a histidine to tyrosine amino acid 600 substitution (codon CAT → TAT base substitution) in gene XDEN_01573 which is encoded on the complementary reverse strand.

The nucleotide sequences of SEQ ID NO:1 to SEQ ID NO:6 are provided in Table 20 in Example 12 and in the accompanying sequence listing. Said nucleotide sequences can be amplified from any *Phaffia rhodozyma* strain of the invention by state of the art molecular DNA techniques, e.g., polymerase chain reaction (PCR). Methods for manipulating the yeast genome, including deletion, insertion, mutation, and tagging by PCR, are described in Gardner & Jaspersen, Methods Mol Biol. 2014;1205. Another example method for the introduction of single or multiple defined point mutations into the genome of yeast is described in Toulmay & Schneiter, Yeast, 2006 Aug;23(11).

The nucleotide sequences of SEQ ID NO:1 to SEQ ID NO:6 can be identified by PCR amplification using suitable primers. For example, primers of SEQ ID NO: 7 and SEQ ID NO: 8 can be used to identify SEQ ID NO:1, primers of SEQ ID NO: 9 and SEQ ID NO: 10 can be used to identify SEQ ID NO:2, primers of SEQ ID NO: 11 and SEQ ID NO: 12 can be used to identify SEQ ID NO:3, primers of SEQ ID NO: 13 and SEQ ID NO: 14 can be used to identify SEQ ID NO:4, primers of SEQ ID NO: 15 and SEQ ID NO: 16 can be used to identify SEQ ID NO:5, and primers of SEQ ID NO: 17 and SEQ ID NO: 18 can be used to identify SEQ ID NO:6. Primer sequences are provided in Table 20 in Example 12 and in the accompanying sequence listing.

In some embodiments, the genome of the yeast strain of the invention comprises at least one, at least two, at least three, at least four, at least five, or at least six of the nucleotide sequences of SEQ ID NO:1, SEQ ID NO:2, SEQ ID NO:3, SEQ ID NO:4, SEQ ID NO:5, and SEQ ID NO:6.

The yeast strain of the invention can be characterized in that it is obtainable from a *Phaffia rhodozyma* strain selected from the following:

AF-01 (deposit NCYC 4293),
AF-02 (deposit NCYC 4294),
AF-03 (deposit NCYC 4337),
AF-04 (deposit NCYC 4336),
AF-05 (deposit NCYC 4345),
AF-06 (deposit NCYC 4344), and
AF-07 (deposit CBS 146150).

In some embodiments, the yeast strain of the invention is a *Phaffia rhodozyma* strain selected from the following:

AF-01 (deposit NCYC 4293),
AF-02 (deposit NCYC 4294),
AF-03 (deposit NCYC 4337),
AF-04 (deposit NCYC 4336),
AF-05 (deposit NCYC 4345),
AF-06 (deposit NCYC 4344), and
AF-07 (deposit CBS 146150).

Preferred yeast strains of the invention are yeast strains that are obtainable from AF-03 (deposit NCYC 4337), AF-04 (deposit NCYC 4336), AF-05 (deposit NCYC 4345), AF-06 (deposit NCYC 4344), or AF-07 (deposit CBS 146150). Especially preferred yeast strains of the invention are yeast strains that are AF-03 (deposit NCYC 4337), AF-04 (deposit NCYC 4336), AF-05 (deposit NCYC 4345), AF-06 (deposit NCYC 4344) or AF-07 (deposit CBS 146150).

The yeast strains according to the invention that are obtainable from AF-03 (deposit NCYC 4337), AF-04 (deposit NCYC 4336), AF-05 (deposit NCYC 4345), AF-06 (deposit NCYC 4344), or AF-07 (deposit CBS 146150) may, for example, be obtained by mutagenesis (as described below in "Methods of obtaining yeast strains") or by intra-strain breeding. Breeding may be, e.g., by spore to spore or spore to cell or cell to cell hybridization. Preferably, after intra-strain breeding (e.g., hybridization), one or more rounds of selection for the strains ability to produce a greater amount of carotenoid when cultivated in a yeast growth medium comprising, e.g., 3% (w/v) or 4% (w/v) or 5% (w/v) carbon

source compared to when cultivated in a yeast growth medium comprising 1% (w/v) carbon source are performed.

In some embodiments, a yeast strain of the invention is, or is obtainable from, any of AF-02 (deposit NCYC 4294), AF-03 (deposit NCYC 4337), AF-04 (deposit NCYC 4336), AF-05 (deposit NCYC 4345), AF-06 (deposit NCYC 4344), and AF-07 (deposit CBS 146150) and it comprises a genome comprising all of the nucleotide sequences of SEQ ID NO:1, SEQ ID NO:2, SEQ ID NO:3, SEQ ID NO:4, SEQ ID NO:5, and SEQ ID NO:6.

Any yeast strain of the invention can be characterized in that it is capable of producing a greater amount of carotenoid when cultivated in a yeast growth medium comprising at least 3% (w/v) carbon source, such as from about 3% (w/v) to about 10% (w/v), or from about 3% (w/v) to about 8% (w/v), or from about 3% (w/v) to about 5% (w/v), or about 3.5% (w/v), about 4% (w/v), about 4.5% (w/v) or about 5% (w/v), or about 6% (w/v), or about 7% (w/v), or about 8% (w/v) carbon source compared to when cultivated in a yeast growth medium comprising 1% (w/v) carbon source.

For example, the yeast strain of the invention is characterized in that it is capable of producing an amount of carotenoid at least 1.1 times greater when cultivated in a yeast growth medium comprising 3% (w/v) carbon source compared to when cultivated in a yeast growth medium comprising 1% (w/v) carbon source.

Preferably, the yeast strain is characterized in that it is capable of producing an amount of carotenoid at least 1.2 times greater when cultivated in a yeast growth medium comprising 3% (w/v) carbon source compared to when cultivated in a yeast growth medium comprising 1% (w/v) carbon source. More preferably, the yeast strain is characterized in that it is capable of producing an amount of carotenoid at least 1.3 times greater when cultivated in a yeast growth medium comprising 3% (w/v) carbon source compared to when cultivated in a yeast growth medium comprising 1% (w/v) carbon source.

In some embodiments, the yeast strain is characterized in that it is capable of producing an amount of carotenoid at least 1.1 times greater when cultivated in a yeast growth medium comprising 5% (w/v) carbon source compared to when cultivated in a yeast growth medium comprising 1% (w/v) carbon source. Preferably, the yeast strain is characterized in that it is capable of producing an amount of carotenoid at least 1.2 times greater when cultivated in a yeast growth medium comprising 5% (w/v) carbon source compared to when cultivated in a yeast growth medium comprising 1% (w/v) carbon source. More preferably, the yeast strain is characterized in that it is capable of producing an amount of carotenoid at least 1.3 times greater when cultivated in a yeast growth medium comprising 5% (w/v) carbon source compared to when cultivated in a yeast growth medium comprising 1% (w/v) carbon source.

As the skilled person will appreciate, the comparisons referred to herein, e.g., cultivation in 1% (w/v) carbon source versus cultivation in 3% (w/v) carbon source, are comparisons of carotenoid production under growing conditions that differ solely in the amounts of carbon source over the same time period. For example, a typical cultivation period for the yeast strain in each case is at least 3 days, at least 4 days, at least 5 days, at least 6 days, at least 8 days, at least 10 days, at least 12 days or at least 13 days, for example 6 days, 6.5 days, 7 days, 7.5 days, 8 days, 8.5 days, 9 days, 9.5 days, 10 days, 10.5 days, 11 days, 11.5 days, 12 days, 12.5 days, 13 days, 13.5 days, 14 days, 14.5 days or 15 days.

The increased production of carotenoids (e.g., astaxanthin) in higher concentrations of carbon source is a remarkable and surprising effect of the strains of the invention because *Phaffia rhodozyma* typically produce maximal amounts of carotenoids at glucose concentrations lower than 2%, which is a typical condition for high scale production of *Phaffia rhodozyma,* as described in US5922560. For example, it is a common observation that the synthesis of carotenoids is particularly low at glucose concentrations greater than 15 g/L, i.e., 1.5% (w/v) glucose. Therefore, yeast strains of the invention are capable of producing high amounts of carotenoids despite fluctuations in carbon source concentration which typically occur in fermentation processes, particularly large-scale fermentation processes. It is also remarkable that this effect in higher carbon source concentrations occurs whilst at the same time the yeast strains produce very high amounts of carotenoids, as demonstrated in the Examples.

In some embodiments, the yeast strain is characterized in that it is capable of producing an amount of at least 7500 μg carotenoid per g of yeast dry weight when cultivated for at least 7.5 days, or for example, 7.5 days, in a yeast growth medium comprising at least 3% (w/v) carbon source, such as from about 3% (w/v) to about 10% (w/v) carbon source. Preferably, the yeast strain is characterized in that it is capable of producing an amount of at least 12000 μg carotenoid per g of yeast dry weight when cultivated for at least 7.5 days, or for example, 7.5 days, in a yeast growth medium comprising at least 3% (w/v) carbon source, such as for example from about 3% (w/v) to about 10% (w/v) carbon source. More preferably, the yeast strain is characterized in that it is capable of producing an amount of at least 18000 μg carotenoid per g of yeast dry weight when cultivated for at least 7.5 days, or for example, 7.5 days, in a yeast growth medium comprising at least 3% (w/v) carbon source, such as for example from about 3% (w/v) to about 10% (w/v) carbon source.

The yeast strain of the invention can also be characterized in that it is capable of accumulating increased total amounts of carotenoids at 7 days, 8 days, 9 days, 10 days, 11 days, 12 days or 13 days cultivation than when cultivated for 6 days, e.g., more than 1.2, preferably more than 1.3, more preferably more than 1.4 times greater total carotenoid amount than when cultivated for 6 days.

For example, the yeast strain of the invention can be characterized in that it is capable of accumulating more

than 1.4 times greater carotenoids (e.g., astaxanthin) from day 6 to day 13 of cultivation in a yeast growth medium comprising from about 3% (w/v) to about 10% (w/v) carbon source.

In accordance with all yeast strains of the invention, the carotenoid produced is preferably at least 50% (w/w) astaxanthin of the total amount of carotenoid produced.

Process for large-scale fermentation of Phaffia strains of the invention

The invention also provides a process for producing carotenoid by large-scale fermentation of a yeast strain of the invention. Generally, the process involves cultivating the yeast strain in a suitable volume of yeast growth medium, before cultivating in a fermenter, harvesting the cell mass and extracting the carotenoid (e.g., astaxanthin). In a large-scale fermentation process, the carbon source concentration is preferably kept low, e.g., to below 5 g/L throughout the fermentation.

In a typical large-scale fermentation process, a loop of yeast strain can be taken from a petri dish and inoculated into a flask (e.g., of 250 ml) containing medium (e.g., 20 ml) supplemented with a suitable amount of yeast extract (e.g. 15 g/L) and carbon source (e.g., 10 g/L). The flask is incubated with agitating at a suitable temperature for a period of time for the yeast to multiply, e.g. 20°C for 24 hr. Subsequently, a portion of this yeast suspension is inoculated into a larger flask (e.g., a 5 L Erlenmeyer flask) containing yeast growth medium (e.g., 500 ml). The larger flask is then incubated with agitating at a suitable temperature for a period of time for the yeast to multiply, e.g. 20°C for 48 to 72 hr. Subsequently, the yeast suspension is seeded into a fermenter (e.g., 7 L or 70 L) containing yeast growth medium. Suitable fermentation start conditions are, for example: 1 v/v aeration, 300 rpm agitation, % dissolved oxygen ($DO_2$) 40, 20°C and pH 5 to 6. Due to the Crabtree effect, in the processes of the prior art, the carbon source (e.g. glucose) solutions are typically applied with fed-batch fermentation as it is necessary to keep the carbon source concentration below 5 g/L (0.5% (w/v)), preferably below 2 g/L (0.2% (w/v)), throughout the fermentation. The pH can be controlled, for example, using ammonia. Dissolved oxygen can be controlled by agitation and airflow to between 40% and 75% saturation. Antifoam can be added as needed. Example 11 provides further details on suitable fermentation processes. After the fermentation broth containing the *Phaffia rhodozyma* cells has reached a desired cell density or carotenoid/astaxanthin content, the cell mass may be harvested.

Examples of suitable small-batch fermentation and fed-batch fermentation processes are described in US 5,922,560, which is incorporated by reference. Other examples of suitable fermentation processes are described, e.g., in Yeast Strain Selection edited by Chandra J. Panchal, 1990, page 140, which is incorporated by reference, or in US4232045, US3617306A, EP 0 237 427 B2, the entire disclosures of which are also incorporated by reference.

All of these fed-batch processes bear the disadvantage that the amount of the carbon source is difficult to keep at a constant level and it may increase considerably in the fermentation suspension for a short time during the feeding steps, i.e., to higher than about 2% (w/v), which leads to a strong Crabtree effect which is responsible for the production of large amounts of unwanted fermentation by-products such as ethanol and acetate, resulting in low biomass, and greatly reduced carotenoid and astaxanthin synthesis. Since the *Phaffia* strains of the invention are significantly more tolerable against higher amounts of carbon sources in the fermentation media, these strains are particularly suited for the large-scale production of astaxanthin. Also, the carbon source applied can be used more efficiently during the production of carotenoids, in particular astaxanthin, when using the *Phaffia* strains of the invention.

Uses of the strains of the invention

Astaxanthin is widely used as a dietary supplement, such as a nutritional supplement, a food supplement, a beverage supplement, and for food additives such as food colorants. Astaxanthin can also be used as feed supplement for egg-producing chickens and for pig feed. Due to its strong antioxidant activity, astaxanthin also has multiple benefits for human health; for example in Parkinson's disease, cardiovascular diseases, inflammation, and skin health; in promoting rehabilitation after heart attack; and in improving the function of the immune system.

Astaxanthin is also used in the cosmetology industry. Astaxanthin can be placed directly on the skin and it minimizes UV damage, the effects of air pollution and other external influences, and it can slow the aging process. Thus, the invention also provides a use of the strains of the invention to produce a cosmetic composition comprising astaxanthin.

Astaxanthin is also used in aquaculture to provide farm-grown fish, such as salmon, trout, crabs, and shrimp, the flesh reddish colour of wild-grown fish. It is generally incorporated into the diet (e.g., aquaculture feed) of farm-grown fish such as salmonids and trout. Thus, the invention also provides a use of the strains of the invention to produce a dye comprising astaxanthin. The dye is suitable for colouring flesh, preferably fish flesh.

In one embodiment, the invention provides a use of the strains of the invention to produce a product comprising astaxanthin. The product may be a dietary supplement, a nutritional supplement, a food supplement, a beverage supplement, a food additive or a feed additive such as a feed colorant; a pharmaceutical composition for human health, for example for the treatment of Parkinson's disease, cardiovascular diseases, in promoting rehabilitation after heart attack,

or for improving the function of the immune system; or a cosmetic composition.

Methods of obtaining yeast strains

The invention also provides a method of obtaining a carotenoid over-producing yeast strain of *Phaffia rhodozyma* comprising:

a) inducing mutation of a yeast strain of *Phaffia rhodozyma* by incubating it under mutagenic conditions, and
b) selecting a carotenoid over-producing yeast strain obtained thereof, wherein said carotenoid over-producing yeast strain selected in step b) is characterized in that it is capable of producing a greater amount of carotenoid when cultivated in a yeast growth medium comprising at least 3% (w/v) carbon source, such as from about 3% (w/v) to about 10% (w/v), or from about 3% (w/v) to about 8% (w/v), or from about 3% (w/v) to about 5% (w/v), or about 3.5% (w/v), or about 4% (w/v), or about 4.5% (w/v), or about 5% (w/v), or about 6% (w/v), or about 7% (w/v), or about 8% (w/v), carbon source compared to when cultivated in a yeast growth medium comprising 1% (w/v) carbon source.

In one embodiment, said carotenoid over-producing yeast strain selected in step b) is a yeast strain according to the invention as defined herein above.

In some embodiments, the yeast strain which is selected comprises a genome comprising a point mutation at position 1540450 in scaffold 79 at gene XDEN_05955 named *crtE.*

In some embodiments, the yeast strain which is selected comprises a genome comprising a point mutation at position 2028187 in scaffold 69 in the upstream region of gene XDEN_04715 named *erg20.*

In some embodiments, the yeast strain which is selected comprises a genome comprising two-point mutations at positions 318703 and 318738 in scaffold 242, upstream to gene XDEN_00738.

In some embodiments, the yeast strain which is selected comprises a genome comprising two-point mutations at positions 855 and 1020 in scaffold 88, downstream to gene XDEN_06229.

In some embodiments, the yeast strain which is selected comprises a genome comprising a deletion of two bases immediately following position 213879 in scaffold 162, at the gene XDEN_00195.

In some embodiments, the yeast strain which is selected comprises a genome comprising two-point mutations at positions 84078 and 84206 in scaffold 24, at the gene XDEN_01573. Whereas the mutation in position 84078 is defined as an intronic mutation, the mutation in position 84206 is an exonic mutation which leads to a histidine to tyrosine amino acid 600 substitution (codon CAT → TAT base substitution) in gene XDEN_01573 which is encoded on the complementary reverse strand.

In preferred embodiments, the yeast strain which is selected comprises a genome comprising a point mutation at position 1540450 in scaffold 79 at gene XDEN_05955 named *crtE,* and in addition a point mutation at position 2028187 in scaffold 69 in the upstream region of gene XDEN_04715 named *erg20.*

In specific embodiments, the yeast strain which is selected comprises a genome comprising at least one, at least two, at least three, at least four, at least five, or at least six of i) to vi) defined in the following:

i) a point mutation at position 2028187 in scaffold 69, at the upstream region of gene XDEN_04715 (i.e., the point mutation referred to in SEQ ID NO:5 according to Table 20);

ii) a point mutation at position 1540450 in scaffold 79 at gene XDEN_05955 (i.e., the point mutation referred to in SEQ ID NO:4 according to Table 20);

iii) two-point mutations at positions 318703 and 318738 in scaffold 242, upstream to gene XDEN_00738 (i.e., the point mutations referred to in SEQ ID NO:1 according to Table 20);

iv) two-point mutations at positions 855 and 1020 in scaffold 88, downstream to gene XDEN_06229 (i.e., the point mutations referred to in SEQ ID NO:2 according to Table 20);

v) a deletion of two bases immediately following position 213879 in scaffold 162, located at the gene XDEN_00195 (i.e., the mutation referred to in SEQ ID NO:3 according to Table 20); and

vi) two-point mutations at positions 84078 and 84206 in scaffold 24, at the gene XDEN_01573 (i.e., the point mutations referred to in SEQ ID NO:6 according to Table 20).

In some embodiments, the yeast strain which is selected comprises a genome comprising all of the mutations

as defined in i) to vi).

Culture collections all over the world (for example ATCC, NCYC and CBS) hold deposits of wild-type *Phaffia rhodozyma.* Mutagenesis is a process that results in genetic changes in yeast cells, and it is applied to increase the genetic variation within the yeast population by increasing mutation frequency during DNA replication. Mutagenesis can be induced by physical agents like ultraviolet (UV) radiation (non-chemical mutagenesis) or by chemical molecules such as ethylmethane sulphonate (EMS) or N-methyl-N'-nitro-N-nitrosoguanidine (NTG) and diethyl sulfate (chemical mutagenesis). Mutagenesis is typically random. To obtain over-producing astaxanthin strains, non-chemical mutagenesis and chemical mutagenesis can be used alone or in combination.

According to a typical mutagenesis protocol (for example in EP0438182A1), a chemical mutagen is added to a cell suspension in buffer, and vials are incubated at room temperature with or without shaking for a duration of time such as between 15 minutes to 2 hours. After exposure to the mutagen, cells are collected, diluted and seeded onto agar medium plates. The composition of plates usually contains a carbon source (such as glucose, raffinose, or others), yeast extract, peptone and agar. The cells are distributed over the plate surface by glass beads of a cell spreader (Drigalskispatel). Seeded plates are inverted and incubated for example at around 20°C for up to 10 days. Similar cultivation procedures can be used for non-chemical mutagenesis.

After each mutagenesis cycle, yeast mutants are screened for the desired property. For example, after each cycle of mutagenesis, yeast cells can be incubated until the appearance of improved pigmentation that can be easily recognized by visual screening. In order to identify strains which overproduce the pigment astaxanthin, selective medium and/or screening agents that strengthen colour differences between colonies can be used.

Plates are visually screened and high pigmentation colonies are picked for an isolation seeding. These isolates are further transferred to flasks (e.g., 250 ml Erlenmeyer flask) with growth medium (routinely containing a carbon source, peptone, and yeast extract) for a period of incubation (e.g. 6-7 days at around 20°C), followed by total carotenoid and astaxanthin analysis. An example of mutagenesis and screening of *Phaffia rhodozyma* is provided in US5922560.

Importantly, the strain has not been modified by recombinant DNA technology.

Isolated strains with highest total carotenoids and astaxanthin levels are used for repeated mutagenesis to obtain strains which show very high astaxanthin concentrations, and which produce a greater amount of carotenoid when cultivated in a yeast growth medium comprising at least 3% (w/v) carbon source, such as from about 3% (w/v) to about 10% (w/v), or from about 3% (w/v) to about 8% (w/v), or from about 3% (w/v) to about 5% (w/v), or about 3.5% (w/v), about 4% (w/v), about 4.5% (w/v) or about 5% (w/v), or about 6% (w/v), or about 7% (w/v), or about 8% (w/v), compared to the amount of carotenoid produced when cultivated in a yeast growth medium comprising 1% (w/v) carbon source. In some embodiments, the ratio of carotenoid produced when cultivated in 3% (w/v) carbon source compared to when cultivated in 1% (w/v) carbon source is at least 1.1, preferably it is at least 1.2, and more preferably it is at least 1.3. In some embodiments, the ratio of carotenoid produced when cultivated in 5% (w/v) carbon source compared to when cultivated in 1% (w/v) carbon source is at least 1.1, preferably it is at least 1.2, and more preferably it is at least 1.3.

In some embodiments, the yeast strain obtained by the method is further characterized in that it is capable of producing an amount of at least 7500 μg carotenoid per g of yeast dry weight when cultivated for at least 7.5 days in a yeast growth medium comprising at least 3% (w/v) carbon source.

Preferably, the yeast strain obtained by the method is further characterized in that it is capable of producing an amount of at least 12000 μg carotenoid per g of yeast dry weight when cultivated for at least 7.5 days in a yeast growth medium comprising at least 3% (w/v) carbon source.

More preferably, the yeast strain obtained by the method is further characterized in that it is capable of producing an amount of at least 18000 μg carotenoid per g of yeast dry weight when cultivated for at least 7.5 days in a yeast growth medium comprising at least 3% (w/v) carbon source.

In some embodiments, the yeast strain obtained by the method is characterized in that it is capable of accumulating increased total amounts of carotenoids at 7 days, 8 days, 9 days, 10 days, 11 days, 12 days or 13 days cultivation than when cultivated for 6 days, e.g., more than 1.2, preferably more than 1.3, more preferably more than 1.4 times greater total carotenoid amount than when cultivated for 6 days.

Accordingly, the invention provides a carotenoid over-producing yeast strain obtained by a method provided herein.

## Carotenoid extraction and analysis

The invention also provides astaxanthin produced by a strain of the invention. In some embodiments, the astaxanthin produced by a strain of the invention is provided in the form of a composition.

Total carotenoid analysis is widely used in the investigation of *Phaffia rhodozyma* over-producing strains and is described in the literature, for example in Sedmak *et.al,* 1990. An example of such a protocol is as follows. Cells are collected and washed, e.g. with de-ionized water. Optionally, cell walls can be disrupted by the enzymatic technique for example as previously described in M. Michelon et.al., 2012. In brief, pelleted cells are incubated with a lysing enzyme

from *Trichoderma harzianum* in 0.2 M sodium acetate buffer pH=5.04 for 30 min at 55°C with agitating, followed by centrifugation and the pellet is washed twice with distilled water. Washed cells are dried, re-suspended in DMSO (pre-heated to around 55°C). The tubes are vortexed vigorously and phosphate buffer and organic solvent are subsequently added. The phases are separated by centrifugation and the organic phase is removed. Samples of the organic phase can be further diluted with DMSO and the OD measured by spectrophotometer at 470-495nm. The total carotenoid content is determined from the absorbency at 470-495nm.

The carotenoid content can be calculated using the following equation.

$$TC = (A/E) \times 10^4 \times \text{dilution factor}$$

A is Value of absorbency
E is Extinction coefficient
(The extinction coefficient E=2150 was determined by using synthetic astaxanthin.)

For astaxanthin extraction and analysis, yeast cells are collected and washed with de-ionized water or sodium acetate buffer (0.2 M, pH=5.04) and pelleted by centrifugation. Optionally, cell walls can be disrupted by the enzymatic technique for example as previously described in M. Michelon et.al., 2012. In brief, pelleted cells are incubated with a lysing enzyme from *Trichoderma harzianum* in 0.2 M sodium acetate buffer pH=5.04 for 30 min at 55°C with agitating. After that, cells are centrifugated and the pellet is washed twice with distilled water. Washed cells are transferred into vessels, pelleted and residual water is removed. Cell pellets are ruptured, e.g., with acetone and glass beads, followed by centrifugation to pellet cell debris and glass beads.

Astaxanthin levels in the obtained solution can be quantified by normal-phase HPLC with a silica column and hexane:acetone (e.g., 86:14) mobile phase. To calculate astaxanthin concentration, a standard curve of synthetic astaxanthin can be used.

## EXAMPLES

Example 1: Development of astaxanthin over-producinq strains

*Phaffia rhodozyma* "AF" strains **AF-01** (NCYC 4293), **AF-02** (NCYC 4294), **AF-03** (NCYC 4337), **AF-04** (NCYC 4336), **AF-05** (NCYC 4345), **AF-06** (NCYC 4344) and **AF-07** (CBS 146150)) over-producing astaxanthin were obtained from a wild-type *Phaffia rhodozyma* strain using classical strain improvement methodology of repeated cycles of mutagenesis and selection.

Example 2: Strain Descriptions: Colony Morphology

Colony morphology of the novel strains was observed after 6 days and 10 days cultivation on standard YM-agar plates. The results are summarized in Table 1.

*Table 1: Colony Morphology*

| **Strain** | **Colony description** | **Colony diameter (mm)** | |
|---|---|---|---|
| | | **6 d** | **10 d** |
| Wild-type | smooth and round colonies and the colour is light orange. Some colony size or colour heterogeneity exists within the culture at a very low level | 1-2.5 | 3-4.5 |
| AF-01 | smooth and round colonies and the colour is orange. Some colony size or colour heterogeneity exists within the culture at a very low level | 1-2 | 2.5-4 |
| AF-02 | smooth and round colonies and the colour is orange. Some colony size or colour heterogeneity exists within the culture at a very low level | 1-2 | 2-4 |
| AF-03 | smooth and round colonies and the colour is red. Some colony size heterogeneity exists within the culture at a very low level | 1-1.8 | 1.5-4 |

(continued)

| Strain | Colony description | Colony diameter (mm) | |
|---|---|---|---|
| | | 6 d | 10 d |
| AF-04 | smooth and round colonies and the colour is red. Some colony size heterogeneity exists within the culture at a very low level | 1-1.5 | 1.5-4 |
| AF-05 | smooth and round colonies and the colour is red. Some colony size heterogeneity exists within the culture at a very low level | 1-2 | 1-4.5 |
| AF-06 | smooth and round colonies and the colour is red. Some colony size heterogeneity exists within the culture at a very low level | 1-1.5 | 1-4 |
| AF-07 | smooth and round colonies and the colour is red. Some colony size heterogeneity exists within the culture at a very low level | ≤1 | 1-4 |
| ATCC 74220 (reference, see US 5,922,560) | some colony size and colour heterogeneity exists within the culture at very high levels. Colour range from yellow to orange | ≤1 | 1-2.5 |

Example 3: High carotenoid production in AF-*Phaffia rhodozyma* mutants growing in media containing 3% (w/v) carbon source

A full inoculation loop of fresh yeast was transferred from a YM agar plate to a 250 ml Erlenmeyer flask containing 20 ml YM media with 3% (w/v) glucose and incubated in a shaker incubator at 20°C with agitating for 48 hours, for each mutant strain. The cell suspensions were aseptically collected and concentrated approximately 2-fold and a volume of 800 μl of concentrated cell suspension was transferred into 250 ml Erlenmeyer flasks containing 20 ml of a medium (peptone 0.5%, yeast extract 0.3%, carbon source 3%, and malt extract 0.3%) and incubated at 20°C for 7.5 days with agitating. The *carotenoids* content of the mutant cultures was measured and results are summarized in Tables 2 and 3.

*Table 2: Carotenoids content of the Phaffia rhodozyma strains*

| Strain | TC production (μg TC / g d.m.) in YM containing 3% glucose |
|---|---|
| AF-02 | 7792 |
| AF-03 | 23142 |

*Table 3: Carotenoids content of the Phaffia rhodozyma strains*

| Strain | TC production (μg TC / g d.m.) in YM containing 3% glucose | TC production (μg TC / g d.m.) in YM containing 1.5% raffinose and 1.5 % glucose |
|---|---|---|
| AF-04 | 18047 | 22968 |
| AF-05 | 18723 | 23721 |
| AF-06 | 20259 | 23187 |

*Table 4: Carotenoids content of the Phaffia rhodozyma strains*

| Strain | TC production (μg TC / g d.m.) in YM containing 1.5% raffinose and 1.5% glucose |
|---|---|
| AF-03 | 23326 |
| AF-07 | 36874 |

Tables 2, 3 and 4 demonstrate that the novel strains according to the invention produce extremely high carotenoids levels, above 7,500 μg TC / g d.m., preferably above 18,000 μg TC / g d.m., and most preferably above 23,000 μg TC / g d.m..

Example 4: Astaxanthin production in AF-Phaffia rhodozyma mutants

A full inoculation loop of fresh yeast was transferred from a YM agar plate to a 250 ml Erlenmeyer flask containing 20 ml YM media with 3% (w/v) glucose and incubated in a shaker incubator at 20°C with agitating for 48 hours, for each mutant strain. The cell suspensions were aseptically collected and concentrated approximately 2-fold and a volume of 800 μl of concentrated cell suspension was transferred into 250 ml Erlenmeyer flasks containing 20 ml of a medium (peptone 0.5%, 1.5% glucose, yeast extract 0.3%, raffinose 1.5%, and malt extract 0.3%) and incubated at 20°C for 7.5 days with agitating. The astaxanthin concentration of the mutant cultures was measured. Results are summarized in Table 5.

*Table 5: Astaxanthin content of the Phaffia rhodozyma strains*

| **Strain** | **Astaxanthin (μg/g d.m.)** |
|---|---|
| AF-02 | 7858 |
| AF-03 | 13510 |
| AF-04 | 15586 |
| AF-05 | 14753 |
| AF-06 | 14065 |
| AF-07 | 19817 |

Table 5 demonstrates that strains according to the invention produce high levels of astaxanthin after 7.5 days of cultivation in YM medium, above 13500 μg astaxanthin / g d.m., even up to 19817 μg astaxanthin / g d.m.

Example 5: Growth of novel AF *Phaffia rhodozyma* mutants in media containing 3% glucose concentration resulted in increased carotenoid production compared to growth in media containing 1% glucose concentration

A full inoculation loop of fresh yeast was transferred from a YM agar plate to a 250 ml Erlenmeyer flask containing 20 ml YM media with 1% glucose and incubated in a shaker incubator at 20°C with agitating for 24 hours. The cell suspension was aseptically collected and concentrated approximately 3-fold (from 20 ml to 6 ml). A volume of 300-400 μl of concentrated cell suspension was transferred into a 250 ml Erlenmeyer flask containing 20 ml fresh YM media with either 1% (w/v) or 3% (w/v) glucose and incubated at 20°C with agitating. Six days later, yeast cells were collected and total carotenoid (TC) content was measured. The ratio between TC production in YM 3% (w/v) vs in YM 1% (w/v) glucose is presented in Table 6.

*Table 6: Ratio of TC levels after 6 days cultivation of Phaffia rhodozyma mutants in YM media supplemented with 1%* (w/v) *or 3%* (w/v) *of glucose*

| **Strain** | **TC production (μg TC / g d.m.) in YM 3% glucose/ TC production (μg / g d.m.) in YM medium 1% glucose** |
|---|---|
| AF-01 | 1.14 |
| AF-02 | 1.33 |
| AF-03 | 1.43 |
| AF-04 | 1.13 |
| AF-05 | 1.36 |
| AF-06 | 1.81 |
| AF-07 | 1.61 |
| ATCC74220 | 0.63 |

Table 6 demonstrates that the "AF" strains according to the invention produce higher levels of carotenoids while growing in YM media with higher (3% (w/v) vs 1% (w/v)) glucose concentration, unlike the reference strain, ATCC74220. The ratio of carotenoid production in YM media with 3% (w/v) glucose compared to in YM media with 1% (w/v) glucose is 0.63 for the reference strain ATCC74220, i.e. much less carotenoid is produced in the higher glucose

concentration. In contrast, the ratio for the strains according to the invention is at least 1.1 (see e.g. AF-01 and AF-04), and for some strains at least 1.3 (see e.g. AF-02, AF-03, and AF-05). In the above table 6, AF-06 and AF-07 showed the highest ratio of 1.81 and 1.61, respectively. In addition to the high ratios, the total carotenoid content is very high for the AF-03, AF-04, AF-05, AF-06 and AF-07 strains after cultivation in YM 3% (w/v) glucose in comparison to YM 1% (w/v) glucose. These characteristics of the strains of the invention are exceptional for carotenoid production in *Phaffia rhodozyma,* which are typically cultivated in low to very low glucose concentrations (for example US5922560).

Example 6: Growth of novel AF *Phaffia rhodozyma* mutants in media containing 3% fructose concentration resulted in increased carotenoid production compared to growth in media containing 1% fructose concentration

A full inoculation loop of fresh yeast was transferred from a YM agar plate to a 250 ml Erlenmeyer flask containing 20 ml YM media with 1% glucose and incubated in a shaker incubator at 20°C with agitating for 24 hours. The cell suspension was aseptically collected and concentrated approximately 3-fold (from 20 ml to 6 ml). A volume of 300-400 μl of concentrated cell suspension was transferred into a 250 ml Erlenmeyer flask containing 20 ml fresh YM media with either 1% (w/v) or 3% (w/v) fructose and incubated at 20°C with agitating. Six days later, yeast cells were collected and total carotenoid (TC) content was measured. The ratio between TC production in YM 3% (w/v) vs in YM 1% (w/v) fructose is presented in Table 7.

*Table 7: Ratio of TC levels after 6 days cultivation of Phaffia rhodozyma mutants in YM media supplemented with 1%* (w/v) *or 3%* (w/v) *of fructose*

| **Strain** | **TC production (μg TC / g d.m.) in YM 3% fructose / TC production (μg / g d.m.) in YM medium 1% fructose** |
|---|---|
| AF-02 | 1.38 |
| AF-03 | 1.44 |
| AF-04 | 1.19 |
| AF-05 | 2.26 |
| AF-06 | 2.02 |
| AF-07 | 2.12 |

Table 7 demonstrates that the "AF" strains according to the invention produce higher levels of carotenoids while growing in YM media with higher (3% (w/v) vs 1% (w/v)) fructose concentration. The ratio of carotenoid production in YM media with 3% (w/v) fructose compared to in YM media with 1% (w/v) fructose for the strains according to the invention is at least 1.1 (see e.g. AF-04), and for some strains at least 1.3 (see e.g. AF-02, and AF-03), and highest ratio of at least 2.0 (see e.g. AF-05, AF-06 and AF-07).

Example 7: Growth of novel AF *Phaffia rhodozyma* mutants in media containing 3% sucrose concentration resulted in increased carotenoid production compared to growth in media containing 1% sucrose concentration

A full inoculation loop of fresh yeast was transferred from a YM agar plate to a 250 ml Erlenmeyer flask containing 20 ml YM media with 1% glucose and incubated in a shaker incubator at 20°C with agitating for 24 hours. The cell suspension was aseptically collected and concentrated approximately 3-fold (from 20 ml to 6 ml). A volume of 300-400 μl of concentrated cell suspension was transferred into a 250 ml Erlenmeyer flask containing 20 ml fresh YM media with either 1% (w/v) or 3% (w/v) sucrose and incubated at 20°C with agitating. Six days later, yeast cells were collected and total carotenoid (TC) content was measured. The ratio between TC production in YM 3% (w/v) vs in YM 1% (w/v) sucrose is presented in Table 8.

*Table 8: Ratio of TC levels after 6 days cultivation of Phaffia rhodozyma mutants in YM media supplemented with 1%* (w/v) *or 3%* (w/v) *of sucrose*

| **Strain** | **TC production (μg TC / g d.m.) in YM 3% sucrose / TC production (μg / g d.m.) in YM medium 1% sucrose** |
|---|---|
| AF-02 | 1.51 |
| AF-03 | 1.26 |

(continued)

| Strain | TC production ($\mu$g TC / g d.m.) in YM 3% sucrose / TC production ($\mu$g / g d.m.) in YM medium 1% sucrose |
|---|---|
| AF-04 | 1.3 |
| AF-05 | 1.42 |
| AF-06 | 2.27 |
| AF-07 | 1.84 |
| ATCC74220 | 0.91 |

Table 8 demonstrates that the "AF" strains according to the invention produce higher levels of carotenoids while growing in YM media with higher (3% (w/v) vs 1% (w/v)) sucrose concentration, unlike the reference strain, ATCC74220. The ratio of carotenoid production in YM media with 3% (w/v) sucrose compared to in YM media with 1% (w/v) sucrose is 0.91 for the reference strain ATCC74220, i.e. much less carotenoid is produced in the higher sucrose concentration. In contrast, the ratio for the strains according to the invention is at least 1.2 (see e.g. AF-03 and AF-04), and for some strains at least 1.4 (see e.g. AF-02, and AF-05), and for some strains at least 1.8 (see e.g. AF-06, and AF-07). In the above table 8, AF-06 and AF-07 showed the highest ratio of 2.27 and 1.84 respectively.

Example 8: Cultivation of novel *Phaffia rhodozyma* mutants in media containing 3% glucose concentration resulted in increased astaxanthin production compared to growth in media containing 1% glucose concentration

A full inoculation loop of fresh yeast was transferred from a YM agar plate to a 250 ml Erlenmeyer flask containing 20 ml YM media with 1% glucose and incubated in a shaker incubator at 20°C with agitating for 24 hours. The cell suspension was aseptically collected and concentrated approximately 3-fold (from 20 ml to 6 ml). A volume of 300-400 $\mu$l of concentrated cell suspension was transferred into a 250 ml Erlenmeyer flask containing 20 ml fresh YM media with 3% (w/v) glucose and incubated at 20°C with agitating. After 2 days, the cell suspension was aseptically collected and concentrated approximately 3-fold (from 20 ml to 6 ml). A volume of 300-400 $\mu$l of concentrated cell suspension was transferred into a 250 ml Erlenmeyer flask containing 20 ml fresh YM or YE media (media containing only yeast extract 5 g/L and glucose) with either 1% (w/v) or 3% (w/v) glucose and incubated at 20°C with agitating for 6 days. After that, yeast cells were collected and astaxanthin levels were determined. Results are summarized in Table 9. The results are displayed as the ratio between astaxanthin production ($\mu$g astaxanthin / g d.m.) in YM or YE medium with 3% (w/v) glucose compared to astaxanthin production ($\mu$g astaxanthin / g d.m.) in YM or YE medium with 1% (w/v) glucose.

*Table 9: Ratio of astaxanthin levels after 6 days cultivation of Phaffia rhodozyma mutants in YM and YE media supplemented with 1%* (w/v) *and 3%* (w/v) *of glucose*

| Strain | Astaxanthin production ($\mu$g / g d.m.) in 3% glucose / Astaxanthin production ($\mu$g / g d.m.) in 1% glucose | |
|---|---|---|
| | YM medium | YE medium |
| AF-05 | 1.51 | 2.01 |
| AF-06 | 1.35 | 3.33 |

Table 9 demonstrates that in two different kinds of media, AF-05 and AF-06 produce higher levels of astaxanthin while growing in media with higher (3% (w/v) vs 1% (w/v)) glucose concentration.

Example 9: Carotenoid production was increased in AF-*Phaffia rhodozyma* mutants growing in media containing 5% glucose or raffinose concentration compared to growth in media containing 1% glucose or raffinose concentration

9.1 A full inoculation loop of fresh yeast was transferred from a YM agar plate to a 250 ml Erlenmeyer flask containing 20 ml YM media with 1% (w/v) glucose and incubated in a shaker incubator at 20°C with agitating for 24 hours. The cell suspension was aseptically collected and concentrated approximately 3-fold (from 20 ml to 6 ml). A volume of 160 $\mu$l of concentrated cell suspension was transferred into a 50 ml TUBESPIN BIOREACTOR containing 8 ml fresh YM media with either 1% or 5% (w/v) glucose and incubated at 20°C at 100 rpm agitating. Six days later, yeast cells were collected and total carotenoid content (TC) was measured. Results are summarized in Table 10.

*Table 10: Ratio of TC levels after 6 days cultivation of Phaffia rhodozyma mutants in YM media supplemented with 1%* (w/v) *or 5%* (w/v) *of glucose*

| **Strain** | **TC production (μg TC / g d.m.) in YM 5% glucose / TC production (μg / g d.m.) in YM medium 1% glucose** |
|---|---|
| AF-01 | 1.3 |
| AF-03 | 1.2 |
| AF-04 | 1.2 |

9.2 In an alternative experiment, a full inoculation loop of fresh yeast was transferred from a YM agar plate to a 250 ml Erlenmeyer flask containing 20 ml YM media with 1% (w/v) glucose and incubated in a shaker incubator at 20°C with agitating for 24 hours. The cell suspension was aseptically collected and concentrated approximately 3-fold (from 20 ml to 6 ml). A volume of 300-400 μl of concentrated cell suspension was transferred into a 250 ml Erlenmeyer flask containing 20 ml fresh YM media with either 1% and 5% (w/v) glucose and incubated at 20°C with agitating. Six days later, yeast cells were collected and total carotenoid (TC) content was measured. Results are summarized in Table 11.

Table 11: *Ratio of TC levels after 6 days cultivation of Phaffia rhodozyma mutants in YM media containing 1% and 5% glucose*

| **Strain** | **TC production (μg TC / g d.m.) in YM 5% glucose / TC production (μg / g d.m.) in YM medium 1% glucose** |
|---|---|
| AF-05 | 1.2 |
| AF-06 | 1.7 |
| AF-07 | 1.5 |

Tables 10 and 11 show that the novel "AF" strains according to the invention produce higher levels of carotenoids while growing in YM media with 5% (w/v) versus 1% (w/v) glucose concentrations.

9.3 In additional experiments a full inoculation loop of fresh yeast was transferred from a YM agar plate to a 250 ml Erlenmeyer flask containing 20 ml YM media with 1% glucose and incubated in a shaker incubator at 20°C with agitating for 24 hours. The cell suspension was aseptically collected and concentrated approximately 3-fold (from 20 ml to 6 ml). A volume of 300-400 μl of concentrated cell suspension was transferred into a 250 ml Erlenmeyer flask containing 20 ml fresh YM media with either 1% or 5% (w/v) raffinose and incubated at 20°C with agitating. Six days later, yeast cells were collected and total carotenoid content (TC) was measured. The ratio between TC production in YM 5% (w/v) vs 1% (w/v) raffinose is presented in Table 12.

*Table 12: Ratio of TC levels after 6 days cultivation of Phaffia rhodozyma mutants in YM media supplemented with 1%* (w/v) *or 5%* (w/v) *of raffinose*

| **Strain** | **TC production (μg TC / g d.m.) in YM 5% raffinose / TC production (μg / g d.m.) in YM medium 1% raffinose** |
|---|---|
| AF-01 | 1.31 |
| AF-02 | 1.59 |
| AF-03 | 1.72 |
| AF-04 | 1.40 |
| AF-05 | 1.34 |
| AF-06 | 1.39 |
| AF-07 | 1.79 |

Example 10: Increase in total carotenoid production in AF-Phaffia rhodozyma mutants in a period of 6 to 13 days

A full inoculation loop of fresh yeast was transferred from a YM agar plate to a 250 ml Erlenmeyer flask containing 20 ml YM media with 1% (w/v) glucose and incubated in a shaker incubator at 20°C with agitating for 24 hours. The cell suspension was aseptically collected and concentrated approximately 3-fold (from 20 ml to 6 ml). A volume of 300-400 μl of concentrated cell suspension was transferred into a 250 ml Erlenmeyer flask containing 20 ml fresh YM media with 3% (w/v) glucose and incubated at 20°C with agitating. After 6-13 days, yeast cells were collected and total carotenoid (TC) content was measured. Results are provided in Table 13 as the ratio to total carotenoid production at day 6.

*Table 13: Ratio of TC levels in AF-Phaffia rhodozyma mutants after 6 to 13 days of cultivation*

| **Strains** | **Ratio of TC production (μg TC / g d.m.) at said day compared to TC production (μg / g d.m.) at day 6** | | | |
|---|---|---|---|---|
| | **7.5 days** | **8.5 days** | **10 days** | **13 days** |
| AF-01 | 1.26 | 1.36 | 1.41 | 1.66 |
| AF-03 | 1.27 | 1.50 | 1.53 | 1.96 |
| AF-04 | 1.27 | 1.30 | 1.56 | 1.58 |

Table 13 demonstrates that the novel strains according to the invention continue to produce and accumulate carotenoids over a period of 6 to 13 days of cultivation.

Example 11: Laboratory-scale fermentation (7 litres)

A loop from frozen culture was transferred onto a petri dish of YM and incubated in 20°C for 72 hr. A loop from the petri dish was inoculated into a 250 ml flask containing 20 ml medium supplemented with 15 g/L yeast extract and 10 g/L glucose. The flask was incubated at 20°C for 24 hr with agitating. Then, 2-7 ml of yeast suspension were inoculated into a 5 L Erlenmeyer flask containing 500 ml of media supplemented with 15 g/L yeast extract and 10 g/L glucose. The Erlenmeyer flask was put into a 20°C incubator with agitation. 48-72 hr later, the yeast suspension was seeded into a 7 L fermenter.

Fermentation start conditions were as follows: 1 vv aeration, 300 rpm agitation, $DO_2$ 40, 20°C and pH 5-6. Glucose solutions were applied with fed-batch fermentation to keep the glucose concentration below 2 g/L, and the pH was controlled using ammonia. Antifoam was added as needed. The medium composition, minerals mix and vitamins mix are provided below.

*Table 14: Typical medium composition*

| | |
|---|---|
| DH2O | 2.3 L |
| Magnesium sulfate | 4.5-8 g |
| Ammonium sulfate | 15 g |
| Yeast extract | 45-60 g |
| Calcium chloride | 0.3-0.9 g |
| Monopotassium phosphate | 4.5-12 g |
| Minerals mix | 3-6 ml |
| Vitamins mix | 30 ml |

*Table 15: Typical minerals mix*

| | |
|---|---|
| DH2O | 1 L |
| Zinc sulfate | 8 g |
| Ferric sulfate | 3 g |
| Copper sulfate | 6 g |
| Manganese sulfate | 3 g |

*Table 16: Typical vitamins mix*

| | |
|---|---|
| DH2O | 100 ml |
| Inositol | 1 g |
| Pyridoxine | 500 mg |
| Thiamin hydrochloride | 150 mg |
| Calcium Panthathonate | 120 mg |
| Biotin | 2 mg |

Pilot-scale fermentation (70 litres)

About 3-4 litres of growth media from the laboratory scale fermenter with 60-80 g growing culture were inoculated into a 70 litre fermenter in a pilot-scale fermentation.

Fermentation start conditions were 1 vv aeration, 300 rpm agitation, DO2 40, 20°C and pH 5-6. The culture was fed glucose as a 60% by weight solution at a rate such that the glucose concentration is less than 5 grams per liter (g/L), preferably less than 2 g/L throughout the fermentation. The glucose concentration at the start of the fermentation was 1.15 g/L. Dissolved oxygen was controlled by agitation and airflow to between 40% and 75% saturation.

*Table 17: Typical pilot medium composition*

| | |
|---|---|
| DH2O | 23 L |
| Magnesium sulfate | 37.5-62.5 g |
| Ammonium sulfate | 125 g |
| Yeast extract | 375-500 g |
| Calcium chloride | 2.5-7.5 g |
| Monopotassium phosphate | 37.5-100 g |
| Minerals mix | 250-500 ml |
| Vitamins mix | 25 ml |

The results of a typical fermentations of AF-06 and AF-07 are presented in Table 18.

*Table 18: TC and Astaxanthin production in 7 L fermenter*

| Strain | Time (hours) | TC μg / g d.m. | Astaxanthin μg / g d.m. |
|---|---|---|---|
| AF-06 | 170 | 19733 | 12372 |
| AF-07 | 160 | 37463 | 20756 |

The results of a typical fermentation of AF-03 are presented in Table 19.

*Table 19: Astaxanthin production in 70 L fermenter*

| Strain | Time (hours) | Astaxanthin μg / g d.m. |
|---|---|---|
| AF-03 | 170 | 16582 |

Table 18 shows that the AF-06 and AF-07 strains produced a high level of total carotenoids and astaxanthin during cultivation in a 7-litre fermenter and Table 19 demonstrates that the AF-03 strain produces a very high level of astaxanthin despite being cultivated in a 70 litre fermenter.

Example 12: The AF-02, AF-03, AF-04, AF-05, AF-06 and AF-07 strains according to the invention were further characterized by Sanger sequencing analysis

The DNA sequences were obtained by using PCR followed by Sanger sequencing using Big-Dye terminator sequencing on a 3730xl capillary electrophoresis device (Applied Biosystems, ThermoFisher Scientific). The PCR used primers designed for amplification of specific genomic DNA fragments as disclosed below. PCR was carried out after preparation of DNA from yeast using standard protocols (DreamTaq Green PCR Master Mix (2X), ThermoFisher Scientific, Waltham, MA), with 1.4 ng DNA under the following conditions: initial denaturation at 95°C for 5 minutes, followed

by 30 cycles of 95°C denaturation for 30 seconds, 55°C or 60°C annealing for 45 seconds, and 72°C elongation for 1 minute.

Table 20 presents the scaffolds, unique sets of primers used and the six amplified genomic DNA fragments present in AF-02, AF-03, AF-04, AF-05, AF-06 and AF-07 strains. All of said strains contain the sequences as set forth in Table 20 - see also the accompanying sequence listing. Mutations and point mutations of the strains of the invention are indicated in bold with underlining.

*Table 20: PCR Results from PCR Amplicons for Genomes of AF-02, AF-03, AF-04, AF-05, AF-06 and AF-07 strains*

| **Scaffold and position** | **Primers** | **Sequence** | **Reference gene annotation** |
|---|---|---|---|
| **Scaffold 242 Accession number: LN483326.1** | Primer 1: CCCACAGAGAGATAAAGAGAGAGA (SEQ ID NO: 7) Primer 2: TACTAGAGGAGAGGAGGTGAGA (SEQ ID NO: 8) | AGGGAACGA**<u>A</u>**ACAGTTGCCGAACGACCATGAAGAAGTGTATATA**<u>A</u>**GAAGCGCCGGAATCAGGTCGTCCATGTTCACTTCTCTCTTTCTCATCCCCTCTCCATCACTGTTCGATTTCAATTCACTTCCTCAATAACTTCCATTCATCCAATGTCCCATTCACAAGGCGATATCGTTGTCTATCCGGATGAAGCACCTCCTCTCTATGATGATCGGCCAT (SEQ ID NO: 1) | Upstream to XDEN_ 00738 |
| **Scaffold 88 Accession number: LN483176.1** | Primer 1: GTTCATCCTTCTACACCACCTAAT (SEQ ID NO: 9) Primer 2: CTCACTCTTTCTACACCACTCTG (SEQ ID NO: 10) | TCATATACAAACATGCTAACGGCGAAGTTAGGCCTCGTATACGGCTCAATCGTTGCAGAGGCCTTTCTGAGGCATCAATACCCTAATGGGCAGAGCCTCATCCAGCGCTCACAAGAGTGCTCTAAAAAGTATAGGGCTCTACTAGCCCCTAAAGGTTTATAAGTAGAGCTTCCATAGCTCTCTAAGGCGCAAAGCCAAAAAGAAGAATAGTATCCTCTTCCAAATCAACAAGCCAATGGCTATTTCTCAAAAGTCCTCTGCGAGGGAGAGCGCTCGACGTCACATGTCCTTTCCGACGCTCTGACGAGTACAATACCTCAATAGCCAATAGAGACCGCTTAAACTTACCAAAGAGGGCTCTAGAGGCTACAAGAGTTTTTCAAGAATGGGGTAACACCCCTCAAAGCGGGGTCCATTACCCCTAGCAAGC | Downstream to XDEN_06229 |

(continued)

| Scaffold and position | Primers | Sequence | Reference gene annotation |
|---|---|---|---|
| | | TAAACAAAAGCAAAGGCGTTCTCTGCAGA GTAAAGCCGAAGCTAAACAAAG**T**TTAAAA GCTAGAGCTTTTCTCAGTACTCAAAGAGC TCAGAGGCTAAGCCTCCTATCTATGCCAT CCAAAAGCAGATAGATAGGAGTGCTTAGA GTGAAAGCTCGTAGAGTACCGCGCACAT GCGCCTTCCTCCCTTACGAGGAAAGCTTT CAAAGTTTAAAAAT**T**TCTTCAAAGCTTTCC GAGAACAATAGCTCTCGGAGCTCAGAGA GCAAGGATAAGCGCTTCTACTCCGTAGA GAGTAGAAAGAGCTCTTTCCTTGCAAAGA CTCCCGCAAAG (SEQ ID NO: 2) | |
| **Scaffold 162 Accession number: LN483249.1** | Primer 1: CACCCAA AGCGTTT AGAA (SEQ ID NO: 11) Primer 2: CATTCCC TGTCCTG AATC (SEQ ID NO: 12) | AAGGTCATATGTTACGATATGTGGCCGTA ACCACATGACACGCGTCGCGTCGCGTGT CCGTCTCGCGCGTCGCGTCTCTGTTGGC CGGTGCGGGCTTGTGTGCGCATGGGTTC GTCGTTCGAGAGAGAGAGAGAGAGAGAG TGGTCCTGCTCGGCGTACATCGATATCAT GATGGCTCA**CATATATATATATATC**ACAC TCGGAAAGAAGATTACAAGGTTTTGGTTC TGTCTCTCTTGATTCTTTCCTCCTCCTCCT TCTTCTTCTCCTTCTTCTTCTCTTTTGTCC TCTTCTTTCCTCCACTTTAGAACCACAAAC TCTTGATGCCTCCTCGGATGGCGTTCCAC CCGGTGGTACCTACAAATGCGGCGTTGT CGATCTTTGTCTGCAATTCATAGTAGTTCT CGAGTCGACGTTTGAGAAAGTCGCGCGT TGACGGCATCGACGGTTCGTTCAACGGA AGCGAGAGCTGGTGAAGCTCAGTGGAGA GGTACAAGAGTGAGAGTGGTAATGACTC GTGCAAGTTCTGAACAGCGTTATGGAGA GATCGCGAGATCGCGAGATTGACAGAGA TACAACGTTAATAGAGTCCTGAAGCTGA (SEQ ID NO: 3) | Intronic mutation at XDEN_00195 |

(continued)

| Scaffold and position | Primers | Sequence | Reference gene annotation |
|---|---|---|---|
| **Scaffold 79 Accession number: LN483167.1** | Primer 1: AAAGACCATTCGCTCACTTCC (SEQ ID NO: 13) Primer 2: CATCCCATCTCTGTGTGTGTTC (SEQ ID NO: 14) | CTTTATCAAGATACGTATCTTTCGAGAACGGAATCTGCGAATTAGGAGTTGACGTGCCCCCGTTTTCAGACGAGGCCGAGGAGGAGGAGGATGCAGAGGAGACGGATGATTGAAGCGAAGCAGATGAAGGAGGAATTACAGGCATGGGTATCG**A**TGTTGGGCGAAGCTTGAAGATCTCTTGATAAGCCAGAAAGTAGACGTAGTTTGCACTATAATCCATAGTAGTTATCCGGT (SEQ ID NO: 4) | Exonic mutation at XDEN_05955 results in Proline to Serine amino acid 181 substitution. |
| **Scaffold 69 Accession number: LN483157.1** | Primer 1: GCAAGGTCCGAGAGTATT (SEQ ID NO: 15) Primer 2: TACAGTGAGCAACCAACA (SEQ ID NO: 16) | ATATGTTCAAA**A**TGTCAGGCGAGTTCTCGGGAGAAAAAAAAAGCGTGGGCTCTGAAACAGTGTGGAAATGTCTACAAAGTGAGCTGGATT (SEQ ID NO: 5) | Upstream to XDEN_04715 |

(continued)

| Scaffold and position | Primers | Sequence | Reference gene annotation |
|---|---|---|---|
| **Scaffold 24 Accession number: <u>LN483116.1</u>** | Primer 1: TGATGGTCAAAGGGAGAAGG (SEQ ID NO: 17)<br>Primer 2: ACAGAGAGCAAGAGCGATAC (SEQ ID NO: 18) | GAAGAAGAGAGCAACATTGGAAGAAACA<br>ACGTTGTCGTCGTCCTGCTCGTCCTCTG<br>GGATTTCGTTCAACTTCTCCTGAAGCATT<br>CTAGCGTAAGTCTCACTAGAAATTTCGAA<br>ATAGCCGCCAGTGCAGAGAGGGAAAGCA<br>GGTAGAATCAGCAAACTAGCACGGTCGA<br>CTAATATAATTGAAAAAAAGAAAGAAA<u>**A**</u>A<br>CACAAAGCTCTCACCTAAGCTCCTCCATC<br>TTCGCAGCTCGATCCTTCTCTGGGTCGTA<br>AGCCAAATGTCTTTTCAAAACCATCGTCT<br>CCCACAAGCTCAAGTGTCGTTTGTGAACG<br>GCGATTCCAT<u>**A**</u>GTATTCTCCAGCCTTACC<br>A (SEQ ID NO: 6) | Intronic mutation at XDEN_01573<br><br>Exonic mutation at XDEN_01573 results in Histidine to Tyrosine amino acid 600 substitution. |

<u>Example 13: Genomic markers of the generated yeast strains were analyzed</u>

Genomic DNA was prepared for sequencing using the Nextera XT kit (Illumina, San Diego, CA) according to the manufacturer's instructions. After processing, libraries were assessed for size using an Agilent TapeStation 2000 automated electrophoresis device (Agilent Technologies, Santa Clara, CA, and for concentration by a Qubit fluorometer (Thermo Fisher Scientific Inc., Waltham, MA. DNA libraries were pooled in equimolar ratio and sequenced using an Illumina NextSeq500 sequencer, with paired-end 2x150 base reads. Bioinformatics analysis was done providing at least 1000 bp long contigs using coverage of at least 250x. Copy number variations were assessed individually for each genome based on coverage, looking for large-scale fluctuations from the genome-wide median.

Strains according to the invention were found to be characterized by point mutations, as described below, in all alleles. In contrast, in the yeast strain CBS6938 used in the systematic sequencing project located at the ENA database [accession ids LN483084-LN483350, (Sharma *et al* 2015)], there are no such point mutations in said genes. All mutations or modifications referred to herein relate to comparison with the *Phaffia rhodozyma* wild type strain.

- Gene XDEN_05955 named *crtE* located at scaffold 79 - comprises a point mutation at position 1540450 (G to A change) leading to amino acid exchange (exchange of Proline to Serine).
- Gene XDEN_04715 named *erg20* located at scaffold 69 - comprises a point mutation at position 2028187 in the upstream region.

<u>Example 14: Percentage of astaxanthin out of total carotenoid content</u>

A full inoculation loop of fresh yeast was transferred from a YM agar plate to a 250 ml Erlenmeyer flask containing 20 ml YM media with 1% glucose and incubated in a shaker incubator at 20°C with agitating for 24 hours. The cell suspension was aseptically collected and concentrated approximately 3-fold (from 20 ml to 6 ml). A volume of 300 to 400 μl of concentrated cell suspension was transferred into a 250 ml Erlenmeyer flask containing 20 ml fresh YM media with 3% (w/v) glucose and incubated at 20°C with agitating. Seven days later, yeast cells were collected, total carotenoid (TC) content and astaxanthin amount were measured. The percentage of astaxanthin out of TC content (w/w) was calculated according to the formula:

$$\% \text{ Astaxanthin} = \text{Astaxanthin amount/TC content} * 100$$

The results are summarized in Table 21.

*Table 21: Percentage of astaxanthin out of TC content (w/w).*

| Strain | % Astaxanthin (w/w) |
|---|---|
| AF-02 | 65% |
| AF-03 | 64% |
| AF-04 | 65% |
| AF-05 | 60% |
| AF-06 | 61% |
| AF-07 | 57% |

As demonstrated in Table 21, astaxanthin is at least 50% (w/w), preferably at least 53% (w/w), preferably at least 55 and most preferably at least 57% (w/w) of the total carotenoids.

## Claims

1. A yeast strain of *Phaffia rhodozyma,* wherein the yeast strain is **characterized in that** it is capable of producing a greater amount of carotenoid when cultivated in a yeast growth medium comprising at least 3% (w/v) carbon source compared to when cultivated in a yeast growth medium comprising 1% (w/v) carbon source.

2. The yeast strain of claim 1, wherein the yeast strain comprises a genome comprising at least one, at least two, at least three, at least four, at least five, or at least six of i) to vi) defined in the following:

   i) a point mutation at position 2028187 in scaffold 69, at the upstream region of gene XDEN_04715 (i.e., the point mutation referred to in SEQ ID NO:5 according to Table 20);
   ii) a point mutation at position 1540450 in scaffold 79 at gene XDEN_05955 (i.e., the point mutation referred to in SEQ ID NO:4 according to Table 20);
   iii) two-point mutations at positions 318703 and 318738 in scaffold 242, upstream to gene XDEN_00738 (i.e., the point mutations referred to in SEQ ID NO:1 according to Table 20);
   iv) two-point mutations at positions 855 and 1020 in scaffold 88, downstream to gene XDEN_06229 (i.e., the point mutations referred to in SEQ ID NO:2 according to Table 20);
   v) a deletion of two bases immediately following position 213879 in scaffold 162, located at the gene XDEN_00195 (i.e., the mutation referred to in SEQ ID NO:3 according to Table 20); and
   vi) two-point mutations at positions 84078 and 84206 in scaffold 24, at the gene XDEN_01573 (i.e., the point mutations referred to in SEQ ID NO:6 according to Table 20).

3. The yeast strain of claims 1 or 2, wherein the yeast strain comprises a genome comprising a point mutation at position 1540450 in scaffold 79 at gene XDEN_05955 named *crtE,* preferably,
   wherein the point mutation at position 1540450 in scaffold 79 at gene XDEN_05955 is a G to A change, resulting in an amino acid exchange of proline to serine, and/or
   wherein the yeast strain comprises a genome comprising a point mutation at position 2028187 in scaffold 69 in the upstream region of gene XDEN_04715 named *erg20.*

4. The yeast strain of any one of the preceding claims, wherein the point mutation(s) is (are) present in all alleles of said gene(s).

5. The yeast strain of any one of the preceding claims, comprising a genome comprising at least one, at least two, at least three, at least four, at least five, or at least six of the nucleotide sequences of SEQ ID NO:1, SEQ ID NO:2, SEQ ID NO:3, SEQ ID NO:4, SEQ ID NO:5, and SEQ ID NO:6.

6. The yeast strain of any one of the preceding claims, wherein the yeast strain is **characterized in that** it is obtainable from a *Phaffia rhodozyma* strain selected from the following:

   AF-01 (deposit NCYC 4293),
   AF-02 (deposit NCYC 4294),
   AF-03 (deposit NCYC 4337),
   AF-04 (deposit NCYC 4336),
   AF-05 (deposit NCYC 4345),
   AF-06 (deposit NCYC 4344), and
   AF-07 (deposit CBS 146150).

7. The yeast strain of any one of the preceding claims, wherein the yeast strain is selected from the following:

   AF-01 (deposit NCYC 4293),
   AF-02 (deposit NCYC 4294),
   AF-03 (deposit NCYC 4337),
   AF-04 (deposit NCYC 4336),
   AF-05 (deposit NCYC 4345),
   AF-06 (deposit NCYC 4344), and
   AF-07 (deposit CBS 146150).

8. The yeast strain of any one of the preceding claims, wherein the yeast strain is **characterized in that** it is capable

of producing a greater amount of carotenoid when cultivated in a yeast growth medium comprising 5% (w/v) carbon source compared to when cultivated in a yeast growth medium comprising 1% (w/v) carbon source.

**9.** Process for producing carotenoid by fermentation of a yeast strain according to any one of the preceding claims.

**10.** A method of obtaining a carotenoid over-producing yeast strain of *Phaffia rhodozyma* comprising:

a) inducing mutation of a yeast strain of *Phaffia rhodozyma* by incubating it under mutagenic conditions, and
b) selecting a carotenoid over-producing yeast strain obtained thereof,

wherein the carotenoid over-producing yeast strain selected in step b) is **characterized in that** it is capable of producing a greater amount of carotenoid when cultivated in a yeast growth medium comprising at least 3% (w/v) carbon source compared to when cultivated in a yeast growth medium comprising 1% (w/v) carbon source;
preferably, said carotenoid over-producing yeast strain selected in step b) is a yeast strain according to any one of claims 1 to 8.

**11.** The yeast strain of any one of claims 1 to 8, the process of claim 9, or the method of claim 10, wherein the carbon source is selected from the group consisting of glucose, fructose, sucrose, maltose, raffinose, xylose, or a combination thereof.

**12.** A carotenoid over-producing yeast strain obtained by the method of claim 10 or claim 11.

**13.** The yeast strain of any one of claims 1 to 8 or 11, the process of claim 9, the method of claim 10 or claim 11, or the carotenoid over-producing yeast strain of claim 12, wherein the carotenoid is at least 50% (w/w) astaxanthin.

**14.** Astaxanthin produced by the yeast strain of any one of claims 1 to 8 or 11.

**15.** Use of the yeast strain of any one of claims 1 to 8, 11 or 13, or of the carotenoid over-producing yeast strain of claim 12 to produce a product comprising astaxanthin, wherein the product is a dietary supplement, a nutritional supplement, a food supplement, a beverage supplement, a food additive or a feed additive such as a feed colorant; a pharmaceutical composition for human health, for example for the treatment of Parkinson's disease, cardiovascular diseases, in promoting rehabilitation after heart attack, or for improving the function of the immune system; or a cosmetic composition.

Europäisches Patentamt
European Patent Office
Office européen des brevets

# EUROPEAN SEARCH REPORT

Application Number
EP 19 21 8701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LILI MIAO ET AL: "Deregulation of phytoene-[beta]-carotene synthase results in derepression of astaxanthin synthesis at high glucose concentration in Phaffia rhodozyma astaxanthin-overproducing strain MK19", BMC MICROBIOLOGY, vol. 19, no. 1, 15 June 2019 (2019-06-15), XP055698968, DOI: 10.1186/s12866-019-1507-6 | 1,10-14 | INV. C12P23/00 C12N9/10 C12N15/01 C12Q1/6895 |
| Y | * abstract; table 1 * * page 2, right-hand column, last paragraph - page 7, left-hand column, paragraph 1; figures 1-5 * ----- | 1-5 | |
| X | GIL-HWAN AN ET AL: "Medium optimization for cultivation of carotenoid hyperproducing Phaffia rhodozyma mutant HT-5F01C", JOURNAL OF FERMENTATION AND BIOENGINEERING., vol. 82, no. 5, 1 January 1996 (1996-01-01), pages 515-518, XP055698437, JP ISSN: 0922-338X, DOI: 10.1016/S0922-338X(97)86996-6 | 14 | TECHNICAL FIELDS SEARCHED (IPC) C12P C12N |
| Y | * abstract * * page 515, right-hand column, last paragraph - page 518, left-hand column, paragraph 1; figures 3,4 * ----- -/-- | 1-5 | |

The present search report has been drawn up for all claims

1

| Place of search | Date of completion of the search | Examiner |
|---|---|---|
| Berlin | 28 May 2020 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 19 21 8701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JENNIFER ALCAÍNO ET AL: "The Involvement of Mig1 from Xanthophyllomyces dendrorhous in Catabolic Repression: An Active Mechanism Contributing to the Regulation of Carotenoid Production", PLOS ONE, vol. 11, no. 9, 1 September 2016 (2016-09-01), pages 1-24, XP055698637, DOI: 10.1371/journal.pone.0162838 | 14 | |
| Y | * abstract; figure 1 * * page 8, paragraph 3 - page 20, paragraph 2; figure 3; tables 1-3 * ----- | 1-5 | |
| X | ANDRÉS MARCOLETA ET AL: ""Glucose and ethanol-dependent transcriptional regulation of the astaxanthin biosynthesis pathway in Xanthophyllomyces dendrorhous"", BMC MICROBIOLOGY, BIOMED CENTRAL LTD, GB, vol. 11, no. 1, 23 August 2011 (2011-08-23), page 190, XP021110046, ISSN: 1471-2180, DOI: 10.1186/1471-2180-11-190 | 14 | |
| Y | * abstract * * page 2, left-hand column, paragraph 4 - page 7, left-hand column, paragraph 1 * ----- -/-- | 1-5 | TECHNICAL FIELDS SEARCHED (IPC) |

The present search report has been drawn up for all claims

1

| Place of search | Date of completion of the search | Examiner |
|---|---|---|
| Berlin | 28 May 2020 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 19 21 8701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br>Y | JENNIFER ALCAÎNO ET AL: "Functional Characterization of the Xanthophyllomyces dendrorhous Farnesyl Pyrophosphate Synthase and Geranylgeranyl Pyrophosphate Synthase Encoding Genes That Are Involved in the Synthesis of Isoprenoid Precursors",<br>PLOS ONE,<br>vol. 9, no. 5, 5 May 2014 (2014-05-05), pages 1-12, XP055698485,<br>DOI: 10.1371/journal.pone.0096626<br>* abstract *<br>* page 7, left-hand column, last paragraph - page 11, left-hand column, paragraph 2; figure 4; table 5 *<br>----- | 14<br><br><br><br><br><br><br><br><br><br><br>1-5 | |
| Y | BARBATO C ET AL: "MILD RIP - AN ALTERNATIVE METHOD FOR IN VIVO MUTAGENESIS OF THE ALBINO-3 GENE IN NEUROSPORA CRASSA",<br>MOLECULAR AND GENERAL GENETICS, SPRINGER VERLAG, BERLIN, DE,<br>vol. 252, no. 4,<br>25 September 1996 (1996-09-25), pages 353-361, XP008001404,<br>ISSN: 0026-8925, DOI: 10.1007/S004380050238<br>* abstract *<br>* page 356, left-hand column, last paragraph - page 359, right-hand column, paragraph 1; figures 1,3A-3C; tables 3,4 *<br>-----<br>-/-- | 1-5 | TECHNICAL FIELDS SEARCHED (IPC) |

The present search report has been drawn up for all claims

1

| Place of search | Date of completion of the search | Examiner |
|---|---|---|
| Berlin | 28 May 2020 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 4

Europäisches Patentamt
European Patent Office
Office européen des brevets

# EUROPEAN SEARCH REPORT

Application Number
EP 19 21 8701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ISABELL SCHMIDT ET AL: "Biotechnological production of astaxanthin with Phaffia rhodozyma/Xanthophyllomyces dendrorhous", APPLIED MICROBIOLOGY AND BIOTECHNOLOGY, SPRINGER, BERLIN, DE, vol. 89, no. 3, 3 November 2010 (2010-11-03), pages 555-571, XP019874946, ISSN: 1432-0614, DOI: 10.1007/S00253-010-2976-6 * the whole document * ----- | 1-15 | |

TECHNICAL FIELDS SEARCHED (IPC)

The present search report has been drawn up for all claims

1

| Place of search | Date of completion of the search | Examiner |
|---|---|---|
| Berlin | 28 May 2020 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5356809 A
- US 5922560 A
- US 4232045 A
- US 3617306 A
- EP 0237427 B2
- EP 0438182 A1


### Non-patent literature cited in the description


- **YANG.** *J Hum Nutr Food Sci,* 2013, vol. 1, 1003
- **AMBATI.** *Mar Drugs.,* January 2014, vol. 12 (1
- **GARDNER ; JASPERSEN.** *Methods Mol Biol.,* 2014, 1205
- **TOULMAY ; SCHNEITER.** *Yeast,* August 2006, vol. 23 (11
- Yeast Strain. 1990, 140

SEQUENCE LISTING

<110> NEXTFERM Technologies Ltd

<120> ASTAXANTHIN OVER-PRODUCING STRAINS OF PHAFFIA RHODOZYMA

<130> NXF19150EP1

<160> 18

<170> BiSSAP 1.3.6

<210> 1
<211> 218
<212> DNA
<213> Phaffia rhodozyma

<220>
<223> Scaffold 242

<400> 1
agggaacgaa acagttgccg aacgaccatg aagaagtgta tataagaagc gccggaatca 60

ggtcgtccat gttcacttct ctctttctca tcccctctcc atcactgttc gatttcaatt 120

cacttcctca ataacttcca ttcatccaat gtcccattca caaggcgata tcgttgtcta 180

tccggatgaa gcacctcctc tctatgatga tcggccat 218

<210> 2
<211> 758
<212> DNA
<213> Phaffia rhodozyma

<220>
<223> Scaffold 88

<400> 2
tcatatacaa acatgctaac ggcgaagtta ggcctcgtat acggctcaat cgttgcagag 60

gcctttctga ggcatcaata ccctaatggg cagagcctca tccagcgctc acaagagtgc 120

tctaaaaagt atagggctct actagcccct aaaggtttat aagtagagct tccatagctc 180

tctaaggcgc aaagccaaaa agaagaatag tatcctcttc caaatcaaca agccaatggc 240

tatttctcaa aagtcctctg cgagggagag cgctcgacgt cacatgtcct ttccgacgct 300

ctgacgagta caatacctca atagccaata gagaccgctt aaacttacca aagagggctc 360

tagaggctac aagagttttt caagaatggg gtaacacccc tcaaagcggg gtccattacc 420

cctagcaagc taaacaaaag caaaggcgtt ctctgcagag taaagccgaa gctaaacaaa 480

gtttaaaagc tagagctttt ctcagtactc aaagagctca gaggctaagc ctcctatcta 540

tgccatccaa aagcagatag ataggagtgc ttagagtgaa agctcgtaga gtaccgcgca 600

catgcgcctt cctcccttac gaggaaagct ttcaaagttt aaaaatttct tcaaagcttt 660

ccgagaacaa tagctctcgg agctcagaga gcaaggataa gcgcttctac tccgtagaga 720

gtagaaagag ctctttcctt gcaaagactc ccgcaaag 758

<210> 3
<211> 601
<212> DNA
<213> Phaffia rhodozyma

<220>
<223> Scaffold 162

<400> 3
aaggtcatat gttacgatat gtggccgtaa ccacatgaca cgcgtcgcgt cgcgtgtccg 60

tctcgcgcgt cgcgtctctg ttggccggtg cgggcttgtg tgcgcatggg ttcgtcgttc 120

gagagagaga gagagagaga gtggtcctgc tcggcgtaca tcgatatcat gatggctcac 180

atatatatat atatcacact cggaaagaag attacaaggt tttggttctg tctctcttga 240

ttctttcctc ctcctccttc ttcttctcct tcttcttctc ttttgtcctc ttctttcctc 300

cactttagaa ccacaaactc ttgatgcctc ctcggatggc gttccacccg gtggtaccta 360

caaatgcggc gttgtcgatc tttgtctgca attcatagta gttctcgagt cgacgtttga 420

gaaagtcgcg cgttgacggc atcgacggtt cgttcaacgg aagcgagagc tggtgaagct 480

cagtggagag gtacaagagt gagagtggta atgactcgtg caagttctga acagcgttat 540

ggagagatcg cgagatcgcg agattgacag agatacaacg ttaatagagt cctgaagctg 600

a 601

<210> 4
<211> 234
<212> DNA
<213> Phaffia rhodozyma

<220>
<223> Scaffold 79

<400> 4
ctttatcaag atacgtatct ttcgagaacg gaatctgcga attaggagtt gacgtgcccc 60

cgttttcaga cgaggccgag gaggaggagg atgcagagga gacggatgat tgaagcgaag 120

cagatgaagg aggaattaca ggcatgggta tcgatgttgg gcgaagcttg aagatctctt 180

gataagccag aaagtagacg tagtttgcac tataatccat agtagttatc cggt 234

<210> 5
<211> 90
<212> DNA
<213> Phaffia rhodozyma

<220>
<223> Scaffold 69

<400> 5
atatgttcaa aatgtcaggc gagttctcgg gagaaaaaaa aagcgtgggc tctgaaacag 60

tgtggaaatg tctacaaagt gagctggatt 90

<210> 6
<211> 345
<212> DNA
<213> Phaffia rhodozyma

<220>
<223> Scaffold 24

<400> 6
gaagaagaga gcaacattgg aagaaacaac gttgtcgtcg tcctgctcgt cctctgggat 60

ttcgttcaac ttctcctgaa gcattctagc gtaagtctca ctagaaattt cgaaatagcc 120

gccagtgcag agagggaaag caggtagaat cagcaaacta gcacggtcga ctaatataat 180

tgaaaaaaag aaagaaaaac acaaagctct cacctaagct cctccatctt cgcagctcga 240

tccttctctg ggtcgtaagc caaatgtctt ttcaaaacca tcgtctccca caagctcaag 300

tgtcgtttgt gaacggcgat tccatagtat tctccagcct tacca 345

<210> 7
<211> 24
<212> DNA
<213> Artificial Sequence

<220>
<223> Scaffold 242 - primer 1

<400> 7
cccacagaga gataaagaga gaga 24

<210> 8
<211> 22
<212> DNA
<213> Artificial Sequence

<220>
<223> Scaffold 242 - primer 2

<400> 8
tactagagga gaggaggtga ga 22

<210> 9
<211> 24
<212> DNA
<213> Artificial Sequence

<220>
<223> Scaffold 88 - primer 1

<400> 9
gttcatcctt ctacaccacc taat 24

<210> 10
<211> 23
<212> DNA
<213> Artificial Sequence

<220>
<223> Scaffold 88 - primer 2

<400> 10
ctcactcttt ctacaccact ctg 23

<210> 11
<211> 18
<212> DNA
<213> Artificial Sequence

<220>
<223> Scaffold 162 - primer 1

<400> 11
cacccaaagc gtttagaa 18

<210> 12
<211> 18
<212> DNA
<213> Artificial Sequence

<220>
<223> Scaffold 162 - primer 2

<400> 12
cattccctgt cctgaatc 18

<210> 13
<211> 21
<212> DNA
<213> Artificial Sequence

<220>
<223> Scaffold 79 - primer 1

<400> 13
aaagaccatt cgctcacttc c 21

<210> 14
<211> 22
<212> DNA

<213> Artificial Sequence

<220>
<223> Scaffold 79 - primer 2

<400> 14
catcccatct ctgtgtgtgt tc 22

<210> 15
<211> 18
<212> DNA
<213> Artificial Sequence

<220>
<223> Scaffold 69 - primer 1

<400> 15
gcaaggtccg agagtatt 18

<210> 16
<211> 18
<212> DNA
<213> Artificial Sequence

<220>
<223> Scaffold 69 - primer 2

<400> 16
tacagtgagc aaccaaca 18

<210> 17
<211> 20
<212> DNA
<213> Artificial Sequence

<220>
<223> Scaffold 24 - primer 1

<400> 17
tgatggtcaa agggagaagg 20

<210> 18
<211> 20
<212> DNA
<213> Artificial Sequence

<220>
<223> Scaffold 24 - primer 2

<400> 18
acagagagca agagcgatac 20